# EUROPEAN PATENT SPECIFICATION

(11) **EP 3 838 885 B1**
(45) Date of publication and mention of the grant of the patent: **29.06.2022**
(21) Application number: 19386056.6
(22) Date of filing: 16.12.2019
(51) Int. Cl.: C12P 7/62

(54) **PROCESS FOR THE PRODUCTION OF OLEOCANTHAL, OLEACEIN AND THEIR ANALOGUES**
VERFAHREN ZUR HERSTELLUNG VON OLEOCANTHAL, OLEACEIN UND DEREN ANALOGA
PROCÉDÉ DE PRODUCTION D'OLÉOCANTHAL, D'OLÉACÉINE ET LEURS ANALOGUES

(43) Date of publication of application: 23.06.2021
(73) Proprietor: National and Kapodistrian University of Athens, 10679 Athens (GR); Pharmagnose S.A., 34100 Eyboia (GR)
(72) Inventor: Skaltsounis, Alexios Leandros, 15127 Ano Melissia (GR); Kostakis, Ioannis, 20300 Loutraki Korinthias (GR); Gaboriaud-Kolar, Nicolas, 34090 Montpellier (FR); Christoforidou, Nikoleta, 14343 Nea Chalkidona (GR); Sarikaki, Georgia, 15772 Zografou, Athens (GR)
(74) Representative: Roukounas, Dimitrios

(56) References cited:
- WO-A2-2018/162769
- ES-A2- 2 693 177
- AMOS B. SMITH ET AL: "Syntheses of (-)-Oleocanthal, a Natural NSAID Found in Extra Virgin Olive Oil, the (-)-Deacetoxy-Oleuropein Aglycone, and Related Analogues", JOURNAL OF ORGANIC CHEMISTRY, vol. 72, no. 18, 1 January 2007 (2007-01-01), pages 6891-6900, XP055548540, US ISSN: 0022-3263, DOI: 10.1021/jo071146k

## Description

### Field of the invention

The present invention relates to a process for the production of certain natural products such as oleocanthal and oleacin as well as their analogues.

### Background of the invention

Extra virgin olive oil, a well-known source of polyphenols, has attracted scientific attention in recent years because of its biological activities and its attribution in many aspects of human health. Although olive oil primarily consists of oleic acid (up to 80 %) and other fatty acids, some minor phenolic compounds, comprising the 1-2 % of the total content, are generally considered to be responsible for the various health benefits of olive oil. The most characteristic compounds in this group are tyrosol (M. Servili, B. Sordini, S. Esposto, S. Urbani, G. Veneziani, I. Di Maio, R. Selvaggini, A. Taticchi, Antioxidants (Basel, Switzerland) 2013, 3, 1-23) and hydroxytyrosol (M. Fuentes de Mendoza, C. De Miguel Gordillo, J. Marin Expoxito, J. Sanchez Casas, M. Martinez Cano, D. Martin Vertedor, M. N. Franco Baltasar, Food Chem. 2013, 141, 2575-2581), the two glucosylated seco-iridoids Oleuropein (G. K. Beauchamp, R. S. J. Keast, D. Morel, J. Lin, J. Pika, Q. Han, C.-H. Lee, A. B. Smith, P. A. S. Breslin, Nature 2005, 437, 45-46) and Ligstroside (K.-L. Pang, K.-Y. Chin, Nutrients 2018, 10, 570); and the two corresponding decarboxymethylated aglycons Oleacein (Y. Gu, J. Wang, L. Peng, Oncol. Rep. 2017, 37, 483-491) and Oleocanthal (M. P. Charalambous, T. Lightfoot, V. Speirs, K. Horgan, N. J. Gooderham, Br. J. Cancer 2009, 101, 106-115).

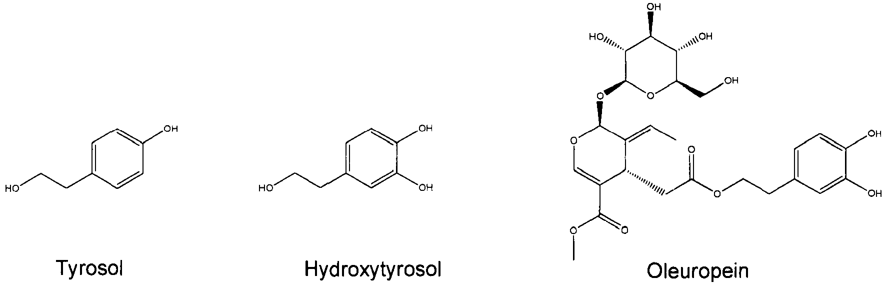

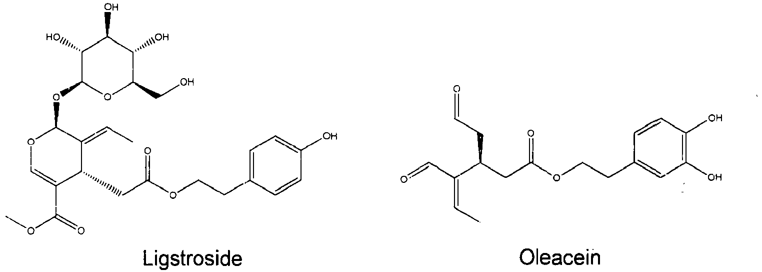

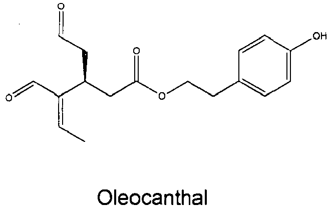

Oleocanthal and oleacein are characterized by a dialdehyde core, connected by an ester moiety with tyrosol and hydroxytyrosol respectively. These two compounds, and especially oleocanthal, have been identified as the agents responsible for the pungency of extra virgin oil. Oleocanthal's recent discovery as COX inhibitor, with similar effect to that of ibuprofen, has dramatically increased its interest both for the study of biological properties but also for the development of new non-steroidal anti-inflammatory drugs (NSAIDS) based on its structure [G. K. Beauchamp, R. S. J. Keast, D. Morel, J. Lin, J. Pika, Q. Han, C.-H. Lee, A. B. Smith, P. A. S. Breslin, Nature 2005, 437, 45-46; K.-L. Pang, K.-Y. Chin, Nutrients 2018, 10, 570]. Additionally, according to many data, oleocanthal demonstrated promising anticancer [Y. Gu, J. Wang, L. Peng, Oncol. Rep. 2017, 37, 483-491; M. P. Charalambous, T. Lightfoot, V. Speirs, K. Horgan, N. J. Gooderham, Br. J. Cancer 2009, 101, 106-115], antimicrobial [E. Medina, A. de Castro, C. Romero, M. Brenes, J. Agric. Food Chem. 2006, 54, 4954-4961], anti-inflammatory [A. Iacono, R. Gomez, J. Sperry, J. Conde, G. Bianco, R. Meli, J. J. Gomez-Reino, A. B. 3rd Smith, O. Gualillo, Arthritis Rheum. 2010, 62, 1675-1682] and neuroprotective activities [H. Qosa, Y. S. Batarseh, M. M. Mohyeldin, K. A. El Sayed, J. N. Keller, A. Kaddoumi, ACS Chem. Neurosci. 2015, 6, 1849-1859; W. Li, J. B. Sperry, A. Crowe, J. Q. Trojanowski, A. B. 3rd Smith, V. M.-Y. Lee, J. Neurochem. 2009, 110, 1339-1351], with no toxic effects. Regarding oleacein, several studies suggest that this compound possesses antimicrobial, anti-proliferative, anti-inflammatory [M. Naruszewicz, M. E. Czerwinska, A. K. Kiss, Curr. Pharm. Des. 2015, 21, 1205-1212; A. Filipek, M. E. Czerwińska, A. K. Kiss, M. Wrzosek, M. Naruszewicz, Phytomedicine 2015, 22, 1255-1261], cardio protective [M. E. Czerwińska, A. K. Kiss, M. Naruszewicz, Food Chem. 2014, 153, 1-8] and antioxidant activity by modulating the Nrf2 pathway [A. Parzonko, M. E. Czerwinska, A. K. Kiss, M. Naruszewicz, Phytomedicine 2013, 20, 1088-1094]: Thus, there is a high demand for these two dialdehydes, in order to initiate more indepth biological studies, however, their low content in olive oil prevents large scale isolation due to apparent high-cost efficiency of the process.

The great interest of these two high-added value natural compounds triggered the development of various synthetic approaches, all involving multi-step total synthesis, with low total yields. Smith's group was the first to report both synthesis and assignment of the absolute configuration of oleocanthal. This 12-steps strategy involved the functionalization of cyclopentanone and its oxidative opening to the dialdehyde corresponding to the natural enantiomer assigning, therefore, its stereochemistry as (-)-oleocanthal [A. B. Smith, Q. Han, P. A. S. Breslin, G. K. Beauchamp, Org. Lett. 2005, 7, 5075-5078]. Later, another approach has been developed based on the opening of a secologanin derivative constructed through a tandem Michael cyclisation/Horner-Woodworth-Emmons olefination. This synthesis, though shorter (9 steps) found limitation due to the lack of control of the stereochemistry during the key cyclisation process leading to the (±)oleocanthal [B. J. English, R. M. Williams, Tetrahedron Lett. 2009, 50, 2713-2715]. A third synthesis has been proposed implying the oxidative opening of a highly functionalized cyclopentane serving as a key intermediate, similar to the one developed in Smith's synthesis, though obtained this time through Sml₂ promoted ring closure [K. Takahashi, H. Morita, T. Honda, Tetrahedron Lett. 2012, 53, 3342-3345]. More recently, an 8-step approach has been proposed, implying an epoxycyclopentanone as key intermediate, though leading to the racemic mixture [M. Valli, E. G. Peviani, A. Porta, A. D'Alfonso, G. Zanoni, G. Vidari, European J. Org. Chem. 2013, 2013, 4332-4336]. Regarding Oleacein, the first total synthesis was accomplished by Smith's group [A. B. Smith, J. B. Sperry, Q. Han, J. Org. Chem. 2007, 72, 6891-6900], and later, a one-step semi-synthesis was accomplished, under Krapcho decarbomethoxylation conditions [K. Vougogiannopoulou, C. Lemus, M. Halabalaki, C. Pergola, O. Werz, A. B. 3rd Smith, S. Michel, L. Skaltsounis, B. Deguin, J. Nat. Prod. 2014, 77, 441-445]. WO 2018/162769 discloses a fully chemical route to oleocanthal and oleacein and their analogues that does not involve an enzymatic transformation.

Thus, there is still a need for an improved process for the production of compounds such as oleocanthal or oleacein and their analogues, which does not have the drawbacks of the processes of the prior art.

### Summary of the invention

The present invention provides a process for the production of compounds such as oleocanthal or oleacein and their analogues starting from oleuropein, an abundantly available product. The process provides in an efficient manner the dialdehyde core of oleocanthal, oleacein and their analogues having the required stereochemistry. Furthermore, the process can be easily adapted for the production of a large number of structurally diverse products by varying the structure of the alcohol moiety in the esterification step. The process involves a small number of steps, is simple, versatile, can be used on a large scale and provides optically pure products.

### Detailed description of the invention

The present invention provides a process for the production of a compound according to Formula (I) 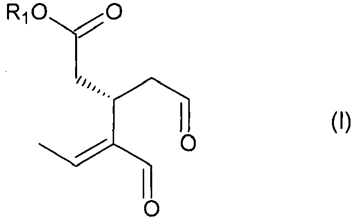 wherein R₁O is an alcohol moiety,
wherein the process comprises the following steps:
a) converting the compound of Formula II 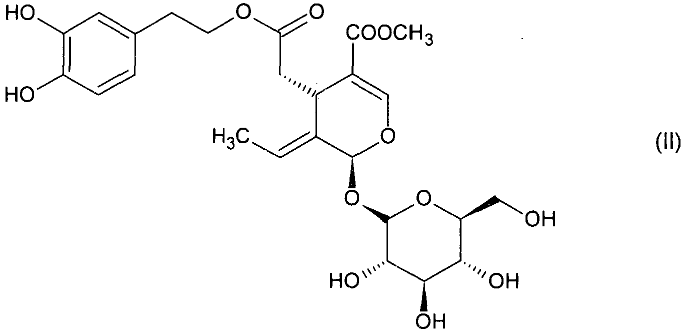 to the compound of Formula III 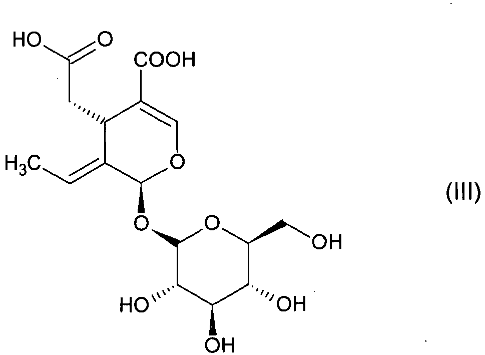
b) converting the compound of Formula III to the compound of Formula IV 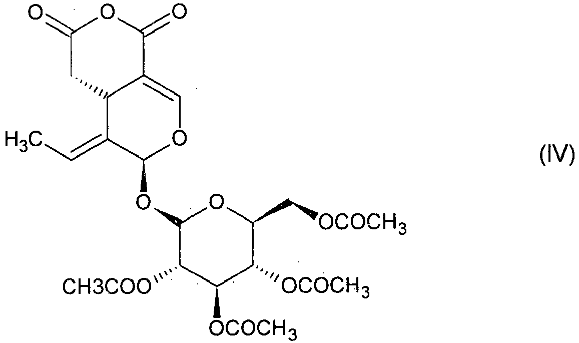
c) converting the compound of Formula IV to the compound of Formula V 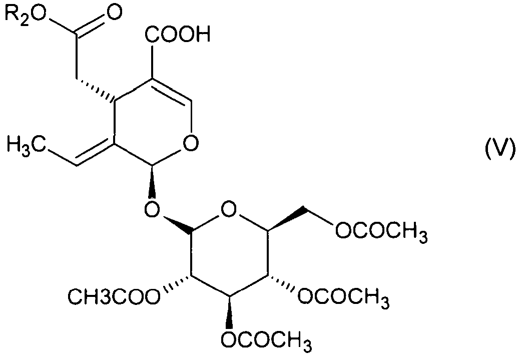 wherein R₂O is the same as R₁O as defined above, or is a protected form of R₁O,
d) converting the compound of Formula V to the compound of Formula VI 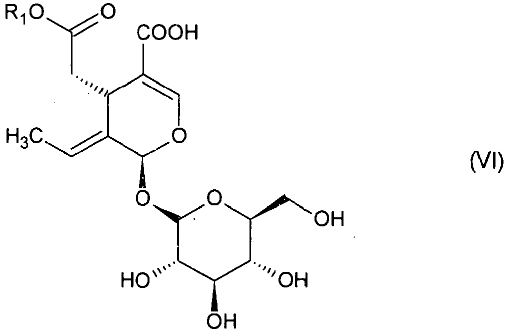 wherein R₁O is as defined above,
e) subjecting the compound of Formula VI to enzymatic hydrolysis with β-glucosidase to give the compound of Formula (I).

The group R₁O in the compound of Formula I can be any alcohol moiety.

Preferably, R₁ is substituted or unsubstituted alkyl or alkene, or a substituted or unsubstituted aryl.

According to a preferred embodiment, R₁ is a C₁-C₁₅ alkyl or alkene, optionally substituted with aryl, alkoxy, acyl, hydroxyl, amino, halogen, thiol, nitrile, carboxyl.

According to another preferred embodiment R₁ is aryl, optionally substituted with alkyl, alkoxy, halogen, amino, hydroxyl, thiol, nitrile, carboxyl.

More preferably, R₁ is 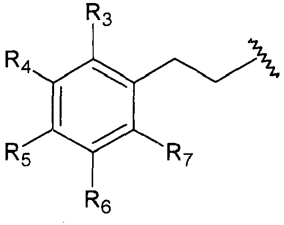 wherein R₃, R₄, R₅, R₆ and R₇ are the same or different and are selected from hydrogen, hydroxyl, halogen, alkyl, alkoxy.

According to another preferred embodiment, R₁ is selected from

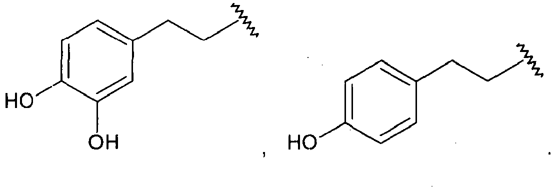

In cases where R1 does not contain any functional groups which can interfere with the esterification reaction of step c), i.e. the reaction of the compound of formula IV with an alcohol of formula R₂OH, R₂ in R₂OH (and in the compound of Formula V) is the same as R₁. In cases where R₁ contains one or more functional groups which can interfere with the esterification reaction, for example a hydroxyl group, this group is preferably in a protected form, for example, as acetyl, benzoyl, benzyl or ether. In such a case R₂ moiety is a protected form of R₁. The protection of functional groups is well known to a person skilled in the art. Thus, for example, in the process for the production of oleocanthal or oleacein, R₁O is a tyrosol or hydrotyrosol moiety respectively as shown below

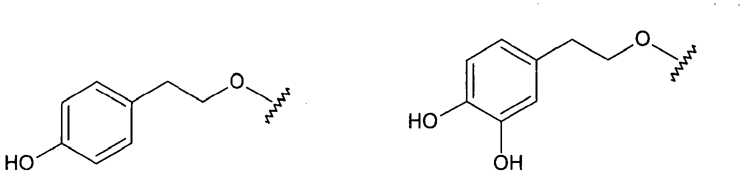

In such a case the mixed anhydride of Formula IV reacts with R₂OH in which the hydroxyl group(s) on the phenyl ring are already protected, for example by an acetyl group. In such a case, R₂ is a protected form of the respective R₁ as shown below

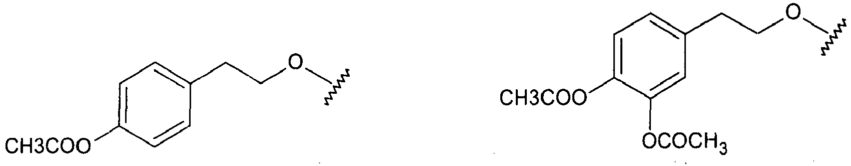

The compound of Formula II is oleuropein, an abundantly available product. Namely, thousands of tons of olive tree leaves are gathered each year in olive oil producing countries, which are burned in the fields, however, they represent a tremendous source of oleuropein, which is the major metabolite that could be found within [N. Xynos, G. Papaefstathiou, M. Psychis, A. Argyropoulou, N. Aligiannis, A.-L. Skaltsounis, J. Supercrit. Fluids 2012, 67, 89-93]. The isolation of oleuropein from olive tree leaves can be carried out using processes well known in the art, such as the one disclosed in V.-I. Boka, A. Argyropoulou, E. Gikas, A. Angelis, N. Aligiannis, A.-L. Skaltsounis, Planta Med. 2015, 81, 1628-1635.

Step a) in the process of the present invention is a saponification, i.e. ester hydrolysis of oleuropein, which can be carried out by processes well know in the art. For example, the reaction can be carried out with NaOH at room temperature.

Alternatively, the saponification of oleuropein can be conducted in an acetonic olive leaf extract, prior the purification, and the compound of Formula III can then be purified for example, by fast centrifugal partition chromatography (FCPC) by using, for example, the system of solvents EtOAc/2-Propanol/EtOH/H₂O/Acetic acid 8/2/1/10/0.5 This sequence provides a simple and powerful approach to obtain high amounts of the compound of Formula III in high optical purity. By following this approach, and depending on the olive leafs used [C. Savournin, B. Baghdikian, R. Elias, F. Dargouth-Kesraoui, K. Boukef, G. Balansard, J. Agric. Food Chem. 2001, 49, 618-621; T. Michel, I. Khlif, P. Kanakis, A. Termentzi, N. Allouche, M. Halabalaki, A.-L. Skaltsounis, Phytochem. Lett. 2015, 11, 424-439.], from 380 g of olive leaves, approximately 4 g of oleoside can be obtained.

One key element in the synthetic procedure of the present invention is the stereoselective mono-esterification of the dicarboxylic acid of Formula III. Attempts to prepare the compound of Formula VI through Yamaguchi esterification conditions gave only small quantities of the desired product, with laborious purifications. The use of many other different conditions did not improve the reaction. Unexpectedly, the present inventors have found that the synthesis of the compound of Formula VI can be achieved through the mixed anhydride of Formula IV. This approach provides, selectively, the desired ester due to steric hindrance around the anhydride moiety. Additionally, the protection of the sugar's hydroxyl groups can be incorporated in one step, during the synthesis of the mixed anhydride.

Step b) involves the synthesis of the mixed anhydride of formula IV. This reaction can be carried out, for example, by treatment of the compound of Formula III with acetic anhydride in the presence of pyridine.

Step c) involves the treatment of the mixed anhydride of Formula IV with a suitable alcohol of formula R₂OH. In cases where R₁ does not contain any functional groups which can interfere with the esterification reaction, R₂ in R₂OH (and in the compound of Formula V) is the same as R₁. In cases where R₁ contains one or more functional groups which can interfere with the esterification reaction, for example a hydroxyl group, this group is preferably in a protected form, for example, as acetyl, benzoyl, benzyl or ether. In such a case, the R₂ moiety is a protected form of R₁. The esterification reaction can be carried out following processes well known in the art, for example, in the presence of triethylamine and 4-dimethylaminopyriding (DMAP) in a suitable solvent, such as chloroform.

Step d) involves the deprotection of the hydroxyl groups of the sugar moiety. This reaction can be carried out, for example, with excess hydrazine or diethylamine in methanol. When R₂ contains protected functional groups the deprotection of these functional groups is preferably carried out simultaneously with the deprotection of the hydroxyl groups of the sugar moiety. Alternatively, the deprotection of these functional groups is carried out separately from the deprotection of the hydroxyl groups of the sugar moiety.

Step e) involves the enzymatic hydrolysis of the compound of Formula VI with β-glucosidase. The reaction can be carried out by following processes well known in the art, such as the one described in S. Hanessian, E. Mainetti, F. Lecomte, Org. Lett. 2006, 8, 4047-4049.

According to a preferred embodiment, steps b) and c) are carried out in one pot, i.e. without isolating the compounds of Formula IV and Formula V respectively. In such a case, the five-step process can be carried out in three pots, providing additional simplicity and advantages.

The process of the present invention provides significant advantages over the processes of the prior art. Thus, it is shorter than the processes of the prior art and provides the final products in good yields without compromising their optical purity. It utilizes oleuropein, an abuntantly available natural product as starting material, it is simple and versatile and it can be carried out on a large scale. Furthermore, it can be easily used for the production of a large number of structurally diverse products by varying the structure of the alcohol moiety.

### Examples

### Example 1

### Oleoside or (4S,5E,6S)-4-(carboxymethyl)-5-ethylidene-6-[(2S,3R,4S,5S,6R)-3,4,5-trihydroxy-6-(hydroxymethyl)oxan-2-yl]oxy-4H-pyran-3-carboxylic acid (compound of Formula III).

A solution of olive leaves acetonic extract (55% oleuropein) (20 g, 20.35 mmol, 1 equiv.) and NaOH (3.25 g, 81.4 mmol, 4 equiv.) in H₂O (150 mL) was stirred for 20 hours at room temperature. The mixture was acidified with 2 N HCI until pH value 4-5 and concentrated under reduced pressure. The purification step took place by using Centrifugal Partition Chromatography (CPC). The biphasic solvent system was composed of ethyl acetate, 2-propanol, ethanol, water and acetic acid in proportion 8/2/1/10/0.5. In a single run of 4 hours on a column with a capacity of 1L, 5.55 g of oleoside, were successfully recovered.

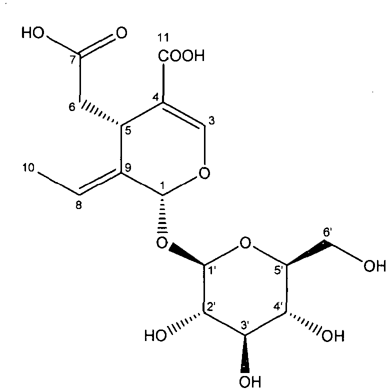

### Example 2

### (2R,4S,E)-3-ethylidene-4-(2-methoxy-2-oxoethyl)-2-(((2R,3S,4R,5S,6S)-3,4,5-triacetoxy-6-(acetoxymethyl)tetrahydro-2H-pyran-2-yl)oxy)-3,4-dihydro-2H-pyran-5-carboxylic acid.

To a solution of the compound of Formula III (400.0 mg, 1.025 mmol, 1 equiv.) in pyridine (805.51 mL, 10.25 mmol, 10 equiv.) at 0 °C was added Ac₂O (1.69 mL, 17.94 mmol, 17.5 equiv.) and stirred at room temperature for 2 h, under argon. The mixture was concentrated under reduced pressure and diluted in dry toluene for azeotropic distillation of pyridine and Ac₂O. Crude compound of Formula IV was diluted in dry ACN, under argon at 0 °C and a mixture of MeOH (45.6 µL, 0.845 mmol, 1.1 equiv.), triethylamine (285.73 µL, 2.05 mmol, 2 equiv.) and DMAP (375.7 mg, 3.07 mmol, 3 equiv.) in dry ACN was added dropwise. After 3 h the reaction mixture was acidified with 2 N HCI until a pH value of 4-5 and concentrated under reduced pressure. Water was added in the crude mixture and washed three times with CH₂Cl₂. The combined organic phases were dried over Na₂SO₄ and concentrated under reduced pressure to afford the title compound which was used for the next step without any further purification.

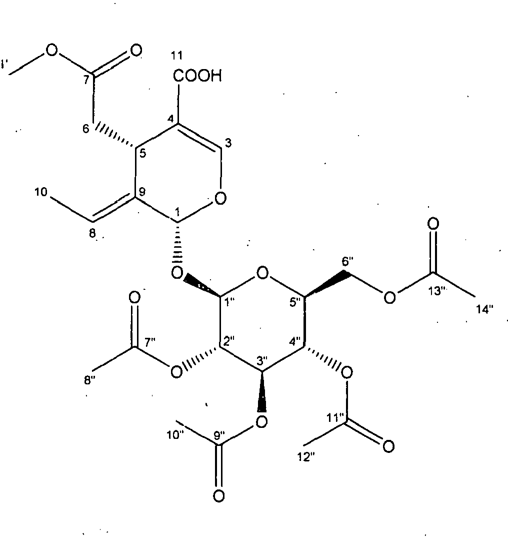

### Example 3

### (2R,4S,E)-3-ethylidene-4-(2-methoxy-2-oxoethyl)-2-(((2R,3S,4R,5R,6S)-3,4,5-trihydroxy-6-(hydroxymethyl)tetrahydro-2H-pyran-2-yl)oxy)-3,4-dihydro-2H-pyran-5-carboxylic acid.

To a stirred solution of crude (2R,4S,E)-3-ethylidene-4-(2-methoxy-2-oxoethyl)-2-(((2R,3S,4R,5S,6S)-3,4,5-triacetoxy-6-(acetoxymethyl)tetrahydro-2H-pyran-2-yl)oxy)-3,4-dihydro-2*H*-pyran-5-carboxylic acid in methanol (2 mL), diethylamine (67.78 µL, 0.875 mmol, 5 equiv.) was added and room temperature for 20 h, under argon. The reaction mixture was acidified with HCI until pH value 4-5 and washed three times with EtOAc, dried over Na₂SO₄, and removed by rotary evaporation. The crude product was purified by silica gel chromatography (CH₂Cl₂/MeOH, 100 → 95:5 v/v) to yield 45 % of the title compound (overall yield from oleoside).

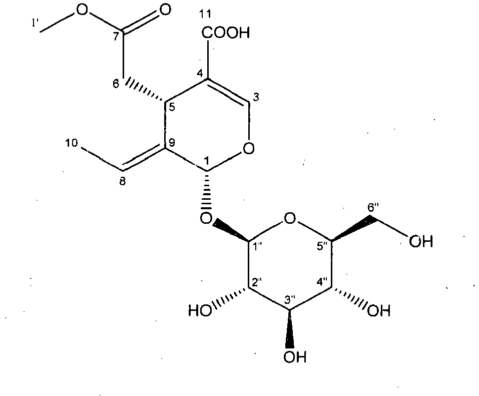

### Example 4

### (S,E)-methyl 4-formyl-3-(2-oxoethyl)hex-4-enoate

To a solution of (2R,4S,E)-3-ethylidene-4-(2-methoxy-2-oxoethyl)-2-(((2R,3S,4R,5R,6S)-3,4,5-trihydroxy-6-(hydroxymethyl)tetrahydro-2H-pyran-2-yl)oxy)-3,4-dihydro-2H-pyran-5-carboxylic acid (50 mg, 0.123 mmol, 1 equiv.) in CH₃COOH/CH₃COONa 0.05M pH=5 (5 mL) β-glucosidase was added (7.09 mg, 17.46 units/mg, 1 equiv.) and stirred at 37 °C for 3 h. The reaction mixture was washed three times with dichloromethane (DCM). The organic layer was dried over Na₂SO₄, and removed by rotary evaporation. The purification was accomplished by silica gel chromatography (DCM/MeOH, 100 → 95:5 v/v) to obtain the title compound (18.2 mg, 75%)

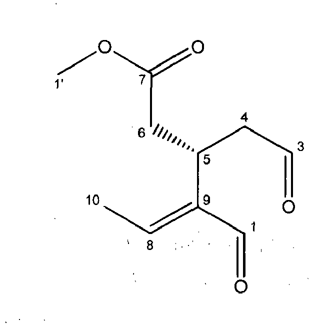

### Example 5

### (2R,4S,E)-4-(2-(4-acetoxyphenethoxy)-2-oxoethyl)-3-ethylidene-2-(((2R,3S,4R,5S,6S)-3.4.5-triacetoxv-6-(acetoxvmethv))tetrahvdro-2H-pvran-2-v!)oxv)-3.4-dihvdro-2H-pyran-5-carboxylic acid.

To a solution of the compound of Formula III (300.0 mg, 0.768 mmol, 1 equiv.) in pyridine (1.27 mL, 15.78 mmol, 20 equiv.) at 0 °C was added Ac₂O (2.54 mL, 26.89 mmol, 35 equiv.) and stirred at room temperature for 20 h. The mixture was concentrated under reduced pressure and diluted in dry toluene for azeotropic distillation of pyridine and Ac₂O. The crude compound of Formula IV was diluted in dry CH₂Cl₂, under argon at 0 °C and a mixture of 4-(2-hydroxyethyl)phenyl acetate (152.31 mg, 0.845 mmol, 1.1 equiv.), triethylamine (321.50 µL, 2.305 mmol, 3 equiv.) and DMAP (28.17 mg, 0.230 mmol, 0.3 equiv.) in dry CH₂Cl₂ was added dropwise. After 20 h the reaction mixture was acidified with HCI 9% until a pH value of 4-5 and washed with H₂O at 0 °C. The water layer was washed three times with EtOAc. The combined organic phases were dried over Na₂SO₄ and concentrated under reduced pressure to afford the title compound which was used for the next step without any further purification.

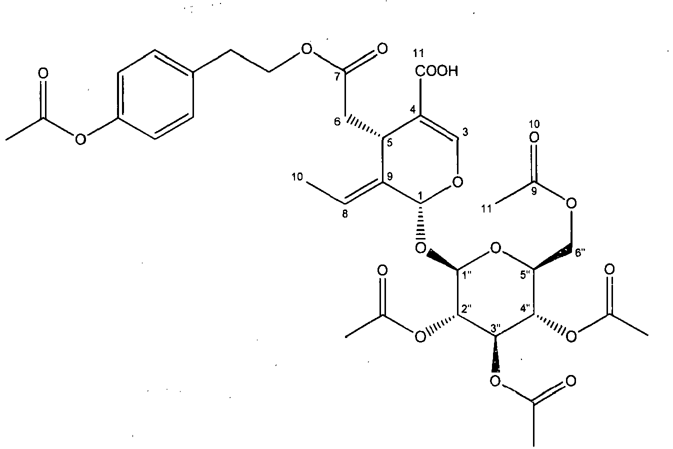

### Example 6

### (2R,4S,E)-3-ethylidene-4-(2-(4-hydroxyphenethoxy)-2-oxoethyl)-2-(((2R,3S,4R,5R,6S)-3,4,5-trihydroxy-6-(hydroxymethyl)tetrahydro-2H-pyran-2-yl)oxy)-3,4-dihydro-2H-pyran-5-carboxylic acid.

To a stirred solution of crude (2R,4S,E)-4-(2-(4-acetoxyphenethoxy)-2-oxoethyl)-3-ethylidene-2-(((2R,3S,4R,5S,6S)-3,4,5-triacetoxy-6-(acetoxymethyl)tetrahydro-2H-pyran-2-yl)oxy)-3,4-dihydro-2H-pyran-5-carboxylic acid in ethanol (85 % aqueous) (5 mL), hydrazine monohydrate (447.28 µL, 9.22 mmol, 12 equiv.) was added and stirred at 44 °C for 6 h. The reaction mixture was acidified with HCI 9% until pH value 4-5 and washed with EtOAc, dried over Na₂SO₄, and removed by rotary evaporation. The crude product was purified by silica gel chromatography (CH₂Cl₂/MeOH, 100 → 85:10 v/v) to yield 40 % of the title compound (overall yield from oleoside).

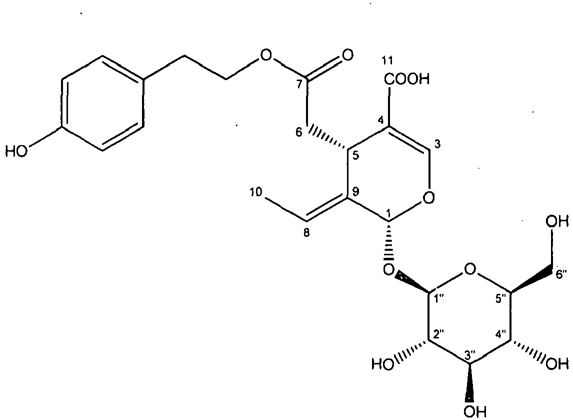

### Example 7

### Oleocantal or (S,E)-4-hydroxyphenethyl 4-formvl-3-(2-oxoethyl)hex-4-enoate

To a solution of (2R,4S,E)-3-ethylidene-4-(2-(4-hydroxyphenethoxy)-2-oxoethyl)-2-(((2R,3S,4R,5R,6S)-3,4,5-trihydroxy-6-(hydroxymethyl)tetrahydro-2H-pyran-2-yl)oxy)-3,4-dihydro-2H-pyran-5-carboxylic acid (50 mg, 0.095 mmol, 1 equiv.) in CH₃COOH/CH₃COONa 0.05M pH=5 (5 mL) β-glucosidase was added (7.09 mg, 17.46 units/mg, 1 equiv.) and stirred at 37 °C for 3 h. The reaction mixture was washed with DCM. The organic layer was dried over Na₂SO₄, and removed by rotary evaporation. The purification was accomplished by silica gel chromatography (DCM/MeOH, 100 → 95:5 v/v) to obtain oleocanthal (21.9 mg, 76%).

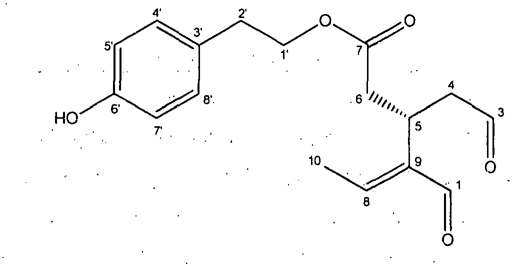

### Example 8

### (2R,4S,E)-4-(2-(3,4-diacetoxyphenethoxy)-2-oxoethyl)-3-ethylidene-2-(((2R,3S,4R,5S,6S)-3,4,5-triacetoxy-6-(acetoxymethyl)tetrahydro-2H-pyran-2-yl)oxy)-3,4-dihydro-2H-pyran-5-carboxylic acid.

To a solution of the compound of Formula III (300.0 mg, 0.768 mmol, 1 equiv.) in pyridine (1.27 mL, 15.78 mmol, 20 equiv.) at 0 °C was added Ac₂O (2.54 mL, 26.89 mmol, 35 equiv.) and stirred at room temperature for 20 h. The mixture was concentrated under reduced pressure and diluted in dry toluene for azeotropic distillation of pyridine and Ac₂O. The crude compound of Formula IV was diluted in dry CH₂Cl₂, under argon at 0 °C and a mixture of 4-(2-hydroxyethyl)-1,2-phenylene diacetate (201.38 mg, 0.845 mmol, 1.1 equiv.), triethylamine (321.50 µL, 2.305 mmol, 3 equiv.) and DMAP (28:17 mg, 0.230 mmol, 0.3 equiv.) in dry:CH₂Cl₂ was added dropwise. After 20 h the reaction mixture was acidified with HCI 9% until a pH value of 4-5 and washed with H₂O at 0 °C. The water layer was washed three times with EtOAc. The combined organic phases were dried over Na₂SO₄ and concentrated under reduced pressure to afford the title compound which was used for the next step without any further purification.

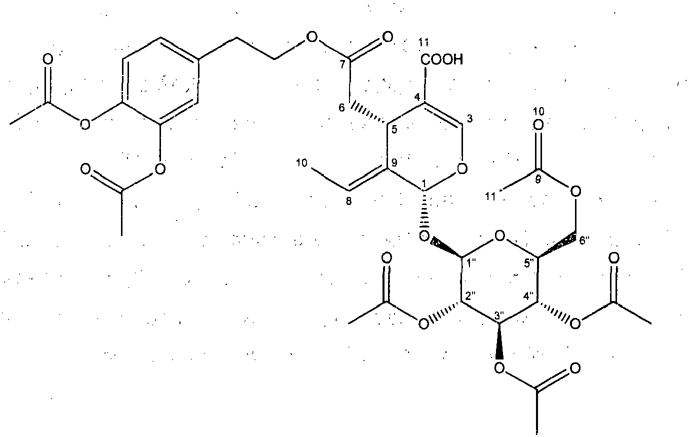

### Example 9

### (2R,4S,E)-4-(2-(3,4-dihydroxyphenethoxy)-2-oxoethyl)-3-ethylidene-2-(((2R,3S,4R,5R,6S)-3,4,5-trihydroxy-6-(hydroxymethyl)tetrahydro-2H-pyran-2-yl)oxy) 3,4-dihydro-2H-pyran-5-carboxylic acid.

To a stirred solution of crude (2R,4S,E)-4-(2-(3,4-diacetoxyphenethoxy)-2-oxoethyl)-3-ethylidene-2-(((2R,3S,4R,5S,6S)-3,4,5-triacetoxy-6-(acetoxymethyl)tetrahydro-2H-pyran-2-yl)oxy)-3,4-dihydro-2H-pyran-5-carboxylic acid in ethanol (85 % aqueous) (5 mL), hydrazine monohydrate (447.28 µL, 9.22 mmol, 12 equiv.) was added and stirred at 44 °C for 6 h. The reaction mixture was acidified with HCI 9 % until pH value 4-5 and washed with EtOAc, dried over Na₂SO4, and removed by rotary evaporation. The crude product was purified by silica gel chromatography (CH₂Cl₂/MeOH, 100 → 85:10 v/v) to yield 46 % of the title compound (overall yield from oleoside).

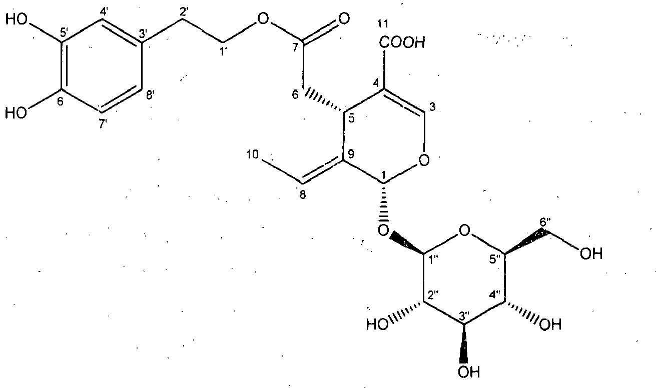

### Example 10

### Oleacein or 2-(3,4-dihydroxyphenyl)ethyl (Z)-4-formyl-3-(2-oxoethyl)hex-4-enoate

To a solution of (2R,4S,E)-4-(2-(3,4-dihydroxyphenethoxy)-2-oxoethyl)-3-ethylidene-2-(((2R,3S,4R,5R,6S)-3,4,5-trihydroxy-6-(hydroxymethyl)tetrahydro-2H-pyran-2-yl)oxy)-3,4-dihydro-2H-pyran-5-carboxylic acid (50 mg, 0.095 mmol, 1 equiv.) in CH₃COOH/CH₃COONa 0.05M pH=5 (5 mL) β-glucosidase was added (7.09 mg, 17.46 units/mg, 1 equiv.) and stirred at 37 °C for 3 h. The reaction mixture was washed with DCM. The organic layer was dried over Na₂SO₄, and removed by rotary evaporation. The purification was accomplished by silica gel chromatography (DCM/MeOH, 100 → 95:5 v/v) to obtain oleacein (22.8 mg, 75%).

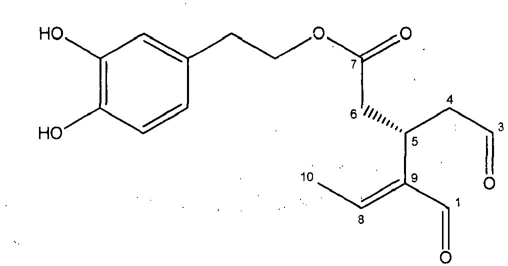

## Claims

1. A process for the production of a compound according to Formula (I) 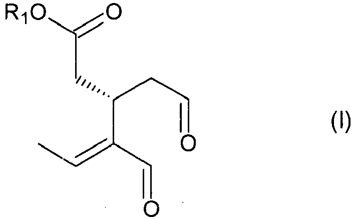 wherein R₁O is an alcohol moiety,
wherein the process comprises the following steps:
a) converting the compound of Formula II 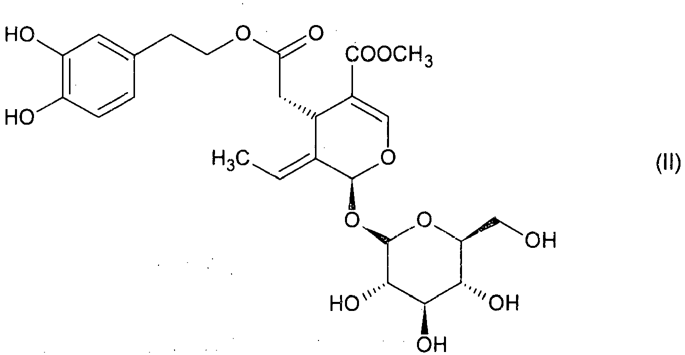 to the compound of Formula III 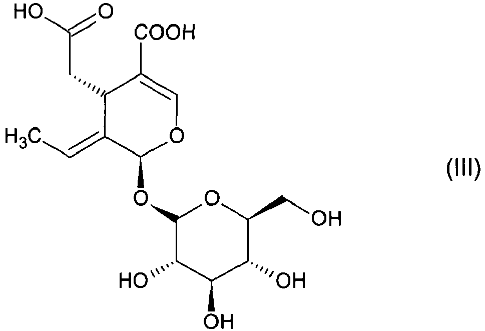
b) converting the compound of Formula III to the compound of Formula IV 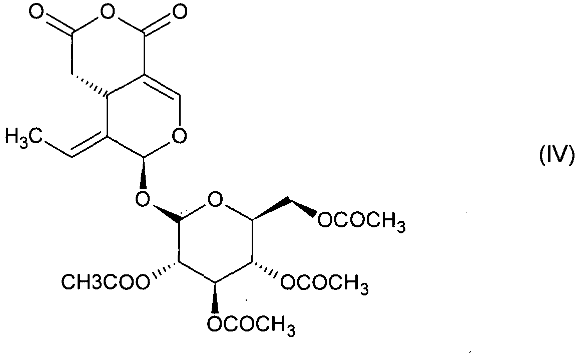
c) converting the compound of Formula IV to the compound of Formula V 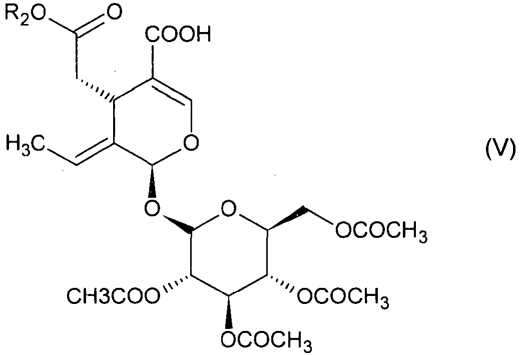 wherein R₂O is the same as R₁O as defined above or is a protected form of R₁O,
d) converting the compound of Formula V to the compound of Formula VI 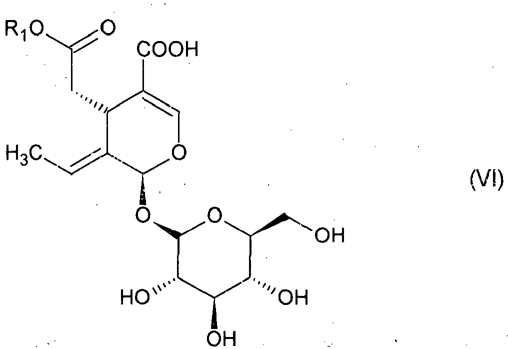 wherein R₁O is as defined above,
e) subjecting the compound of Formula VI to enzymatic hydrolysis with β-glucosidase to give the compound of Formula (I).

2. The process according to claim 1, wherein R₁ is substituted or unsubstituted alkyl or alkene, or a substituted or unsubstituted aryl.

3. The process according to claim 2, wherein R₁ is a C₁-C₁₅ alkyl or alkene, optionally substituted with aryl, alkoxy, acyl, hydroxyl, amino, halogen, thiol, nitrile, carboxyl.

4. The process according to claim 2, wherein R₁ is aryl, optionally substituted with alkyl, alkoxy, halogen, amino, hydroxyl, thiol, nitrile, carboxyl.

5. The process according to claim 4, wherein R₁ is 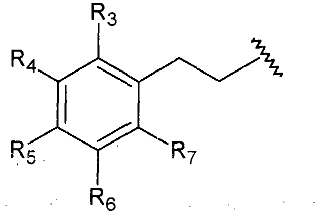 wherein R₃, R₄, R₅, R₆ and R₇ are the same or different and are selected from hydrogen, hydroxyl, halogen, alkyl, alkoxy.

6. The process according to claim 5, wherein R₁ is selected from

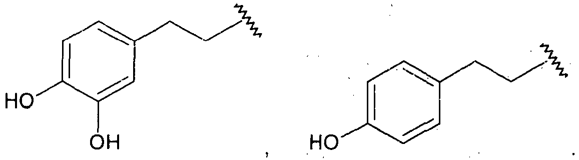

7. The process according to claim 6, wherein R₂O is selected from

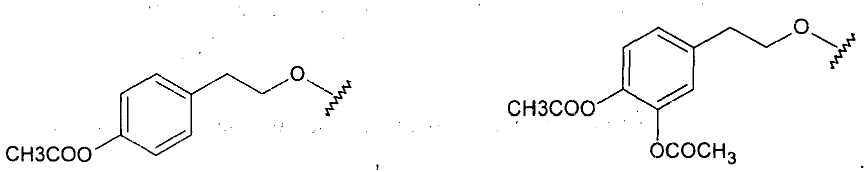

8. The process according to any one of the preceding claims, wherein step a) is carried out with NaOH in an acetonic olive leaf extract.

9. The process according to any one of the preceding claims, wherein step b) is carried out with acetic anhydride in the presence of pyridine.

10. The process according to any one of the preceding claims, wherein step c) is carried out with an alcohol of formula R₂OH in the presence of triethylamine and 4-dimethylaminopyridine (DMAP), wherein R₂O is as defined above.

11. The process according to any one of the preceding claims, wherein step d) is carried out with hydrazine or diethylamine in methanol.

12. The process according to any one of the preceding claims, wherein steps b) and c) are carried out without isolating the compounds of Formula IV and Formula V.

## Patentansprüche

1. Verfahren für die Herstellung einer Verbindung der Formel (I) entsprechend 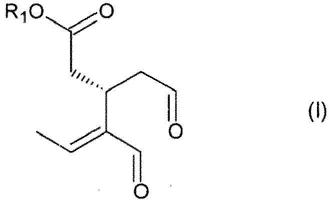 wobei R₁O ein Alkoholanteil ist,
wobei das Verfahren die folgenden Schritte umfasst:
a) Umwandeln der Verbindung der Formel II 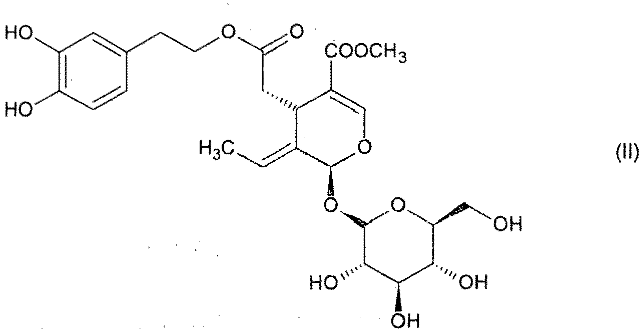 in die Verbindung der Formel III 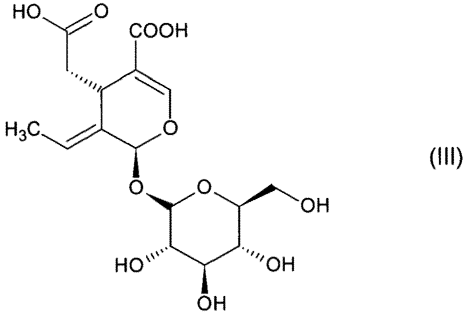
b) Umwandeln der Verbindung der Formel III in die Verbindung der Formel IV 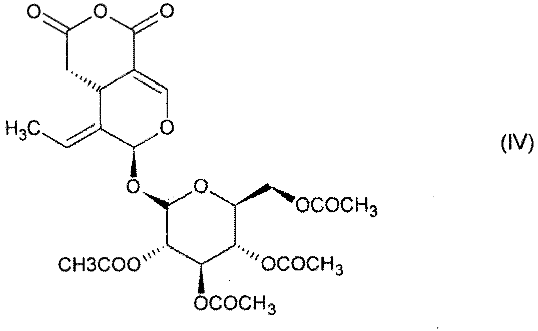
c) Umwandeln der Verbindung der Formel IV in die Verbindung der Formel V 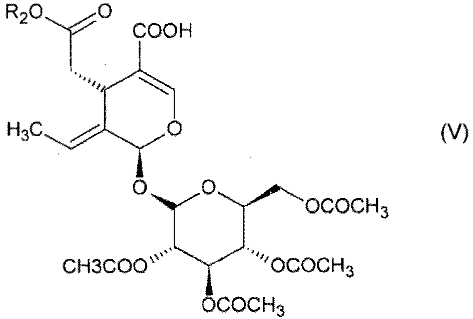 wobei R₂O gleich wie R₁O, wie oben definiert, ist oder eine geschützte Form von R₁O ist
d) Umwandeln der Verbindung der Formel V in die Verbindung der Formel VI 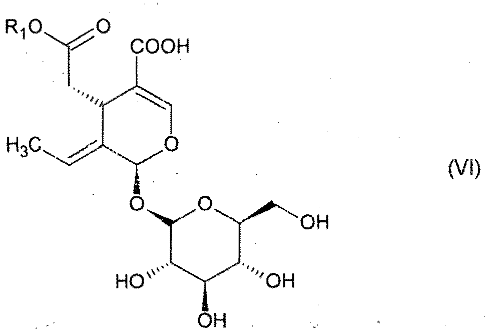 wobei R₁O die obige Definition aufweist,
e) Unterwerfen der Verbindung der Formel VI einer enzymatischen Hydrolyse mit ß-Glukosidase, um die Verbindung der Formel (I) zu ergeben.

2. Verfahren nach Anspruch 1 wobei R₁ substituiertes oder unsubstituiertes Alkyl oder Alken oder ein substituiertes oder unsubstituiertes Aryl ist.

3. Verfahren nach Anspruch 2, wobei R₁ ein C₁-C₁₅-Alkyl oder -Alken ist, das wahlweise mit Aryl, Alkoxy, Acyl, Hydroxyl, Amino, Halogen, Thiol, Nitril, Carboxyl substituiert ist.

4. Verfahren nach Anspruch 2, wobei R₁ Aryl ist, das wahlweise mit Alkyl, Alkoxy, Halogen, Amino, Hydroxyl, Thiol, Nitril, Carboxyl substituiert ist.

5. Verfahren nach Anspruch 4, wobei R₁ 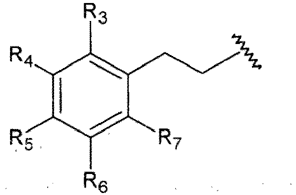 ist, wobei R₃, R₄, P₅, R₆ und R₇ gleich oder verschieden sind und unter Wasserstoff, Hydroxyl, Halogen, Alkyl, Alkoxy ausgewählt sind.

6. Verfahren nach Anspruch 5, wobei R₁ ausgewählt ist von

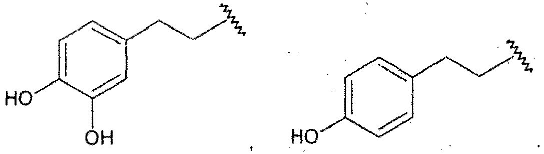

7. Verfahren nach Anspruch 6, wobei R₂O ausgewählt ist von

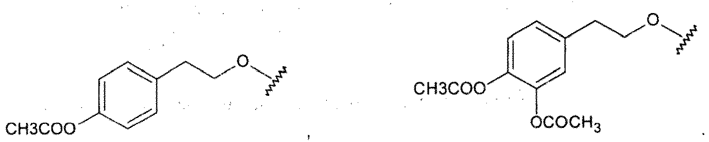

8. Verfahren nach einem der vorhergehenden Ansprüche, wobei der Schritt a) mit NaOH in einem acetonischen Olivenblattextrakt ausgeführt wird.

9. Verfahren nach einem der vorhergehenden Ansprüche, wobei der Schritt b) mit Essigsäureanhydrid in Gegenwart von Pyridin ausgeführt wird.

10. Verfahren nach einem der vorhergehenden Ansprüche, wobei der Schritt c) mit einem Alkohol der Formel R₂OH in Gegenwart von Triethylamin und 4-Dimethylaminopyridin (DMAP) ausgeführt wird, wobei R₂OH die obige Definition aufweist.

11. Verfahren nach einem der vorhergehenden Ansprüche, wobei der Schritt d) mit Hydrazin oder Diethylamin in Methanol ausgeführt wird.

12. Verfahren nach einem der vorhergehenden Ansprüche, wobei die Schritte b) und c) ohne Isolieren der Verbindungen der Formel IV und der Formel V ausgeführt werden.

## Revendications

1. Procédé de production d'un composé de formule (I) 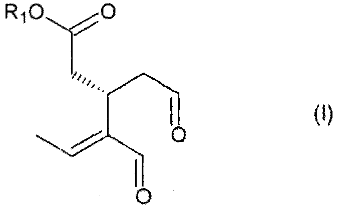 dans lequel R₁O est une fraction alcool,
dans lequel le procédé comprend les étapes suivantes:
a) la conversion du composé de formule II 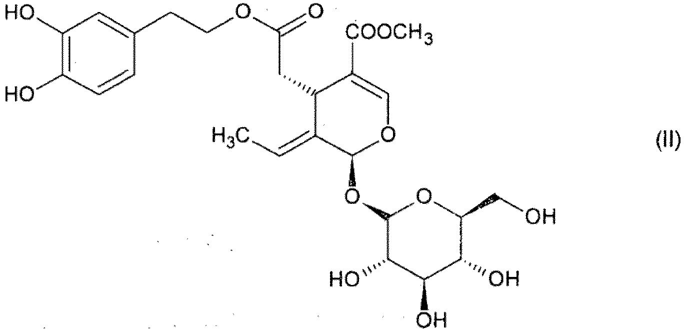 en le composé de formule III 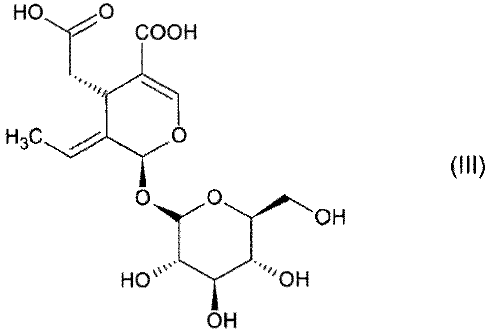
b) la conversion du composé de formule III en le composé de formule IV 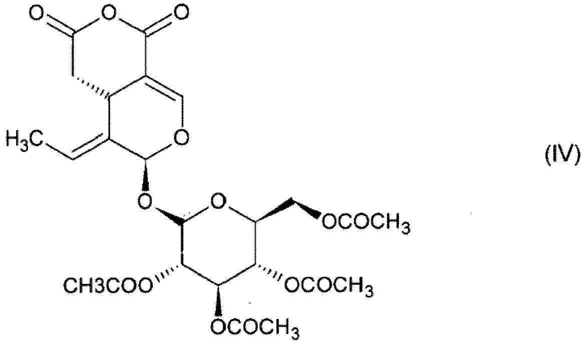
c) la conversion du composé de formule IV en le composé de formule V 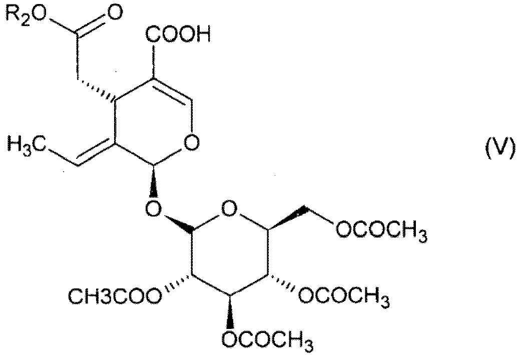 dans lequel R₂O est identique à R₁O tel que défini ci-dessus ou est une forme protégée de R₁O,
d) la conversion du composé de formule V en le composé de formule VI 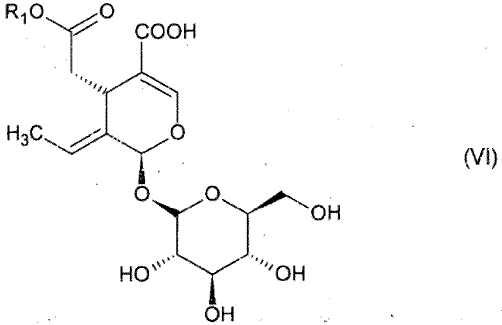 dans lequel R₁O est tel que défini ci-dessus,
e) la soumission du composé de formule VI à une hydrolyse enzymatique avec de la β-glucosidase pour donner le composé de formule (I).

2. Procédé selon la revendication 1, dans lequel R₁ est un alkyle ou alcène substitué ou non substitué ou un aryle substitué ou non substitué.

3. Procédé selon la revendication 2, dans lequel R₁ est un alkyle ou alcène en C₁-C₁₅, facultativement substitué par un aryle, un alcoxy, un acyle, un hydroxyle, un amino, un halogène, un thiol, un nitrile, un carboxyle.

4. Procédé selon la revendication 2, dans lequel R₁ est un aryle, facultativement substitué par un alkyle, un alcoxy, un halogène, un amino, un hydroxyle, un thiol, un nitrile, un carboxyle.

5. Procédé selon la revendication 4, dans lequel R₁ est 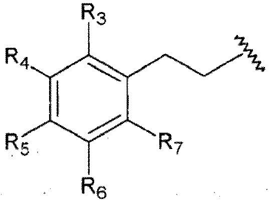 dans lequel R₃, R₄, P₅, R₆ et R₇ sont identiques ou différents et sont sélectionnés parmi un hydrogène, un hydroxyle, un halogène, un alkyle, un alcoxy.

6. Procédé selon la revendication 5, dans lequel R₁ est sélectionné parmi

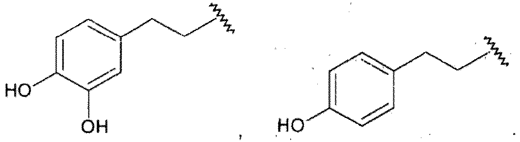

7. Procédé selon la revendication 6, dans lequel R₂O est sélectionné parmi

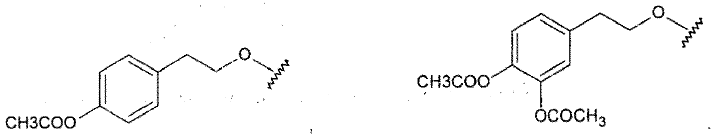

8. Procédé selon l'une quelconque des revendications précédentes, dans lequel l'étape a) est réalisée avec du NaOH dans un extrait acétonique de feuilles d'olivier.

9. Procédé selon l'une quelconque des revendications précédentes, dans lequel l'étape b) est réalisée avec un anhydride acétique en présence de pyridine.

10. Procédé selon l'une quelconque des revendications précédentes, dans lequel l'étape c) est réalisée avec un alcool de formule R₂OH en présence de triéthylamine et de 4-diméthylaminopyridine (DMAP), dans lequel R₂O tel que défini ci-dessus.

11. Procédé selon l'une quelconque des revendications précédentes, dans lequel l'étape d) est réalisée avec de l'hydrazine ou de la diéthylamine dans du méthanol.

12. Procédé selon l'une quelconque des revendications précédentes, dans lequel les étapes b) et c) sont réalisées sans isoler les composés de formule IV et de formule V.
